# EUROPEAN PATENT APPLICATION

(11) **EP 4 772 611 A1**
(43) Date of publication of application: **08.07.2026**
(21) Application number: 24865548.2
(22) Date of filing: 13.09.2024
(51) Int. Cl.: C12N 5/07, A23L 5/00, C12N 1/00

(54) **COMPOSITION FOR BASAL MEDIUM FOR ANIMAL CELL CULTURE**

(30) Priority: 15.09.2023 JP 2023150528
(71) Applicant: TableMark Co., Ltd., Tokyo 104-0045 (JP); IntegriCulture Inc., Tokyo 113-0033 (JP)
(72) Inventor: FUJIE, Nozomi, Tokyo 144-0042 (JP); ABE, Ryosuke, Tokyo 144-0042 (JP); HATANO, Hiroaki, Fujisawa-shi, Kanagawa 251-8555 (JP); TANAKA, Keita, Fujisawa-shi, Kanagawa 251-8555 (JP)
(74) Representative: Isarpatent
(86) International application number: PCT/JP2024/032813
(87) International publication number: WO 2025/058049

(57) **Abstract**

The present invention relates to a basal medium composition for animal cells. The basal medium composition for animal cells according to the present invention contains a yeast extract, amino acids, an inorganic salt, and a sugar, wherein the amino acids in the composition include cystine and/or cysteine, and glutamine.

## Description

### TECHNICAL FIELD

The present invention relates to a basal medium composition for animal cell culture and its use, a method for culturing animal cells, and the like.

### BACKGROUND ART

Cell culture is the process of proliferating and maintaining cells isolated from living tissue in a culture medium. In recent years, research into *in vitro* culture, particularly of animal cells, has progressed, for example into the development of cultured meat. Media used to culture animal cells are generally composed of inorganic salts, carbohydrates, amino acids, vitamins, proteins, peptide fatty acids, lipids, serum, and others.

"Cellular agriculture" is a technology that proliferates cells taken from animals or plants by directly feeding them nutrients, and then produces them as foods. Meat, fish, and other foods produced using cellular agriculture technology are collectively referred to as "cellular foods." Cellular foods that correspond to "meat" are sometimes referred to as "cultured meat."

Cellular foods are being developed worldwide from the perspective of food security and reducing environmental impact. Two particularly important factors for the commercialization of cellular foods are that the ingredients and production process are suitable for food production, and that production costs are reduced. IntegriCulture Inc. (Bunkyo-ku, Tokyo, Japan) succeeded in the research and development of I-MEM 1.0, which reproduces a basal medium composition, the most important element in the production of cellular foods, using only food additive ingredients approved as foods (Press Release PRTIMES "IntegriCulture starts accepting orders worldwide from Wednesday, October 26th for I-MEM, a composition for basal medium made entirely from food ingredients, developed independently by IntegriCulture"; https://prtimes.jp/main/html/rd/p/000000025.000034252.html).

The I-MEM 1.0 brings about the turning of medium components into foods and the reduction of production costs, which have been significant challenges in the production of cell-cultured meat and other products. However, even I-MEM 1.0 is not sufficient in terms of reducing production costs, and there is a need for the development of cheaper and safer compositions for basal media.

Japanese Translation of PCT International Application Publication No. 2005-532057 describes an animal protein-free medium for culturing cells. Claims 1 and 2 of Japanese Translation of PCT International Application Publication No. 2005-532057 are as follows:
[Claim 1] An animal protein free cell culture medium comprising soy hydrolysate and yeast hydrolysate.
[Claim 2] The animal protein-free cell culture medium according to claim 1, wherein the soy hydrolysate is present in a concentration of at least 0.05% (w/v) and yeast hydrolysate is present in a concentration of at least 0.05% (w/v).

As understood from the descriptions of claims 1 and 2, the medium in Japanese Translation of PCT International Application Publication No. 2005-532057 contains the soybean hydrolysate as an essential ingredient. Furthermore, the main purpose of the cell culture in Japanese Translation of PCT International Application Publication No. 2005-532057 is to produce biological products such as viruses and recombinant proteins in the cells (for example, claim 23, paragraphs [0011] and [0012] etc., of Japanese Translation of PCT International Application Publication No. 2005-532057).

There has been a need for the development of a medium for animal cell culture that can be used as a food, reduces the use of food additives and the like while maintaining culturability, and has a reduced environmental impact.

### CITATION LIST

### PATENT LITERATURE

PTL 1: Japanese Translation of PCT International Application Publication No. 2005-532057

### NON PATENT LITERATURE

NPL 1: Press Release PRTIMES "IntegriCulture starts accepting orders worldwide from Wednesday, October 26th for I-MEM, a basal medium composition made entirely from food ingredients, developed independently by IntegriCulture"; https://prtimes.jp/main/html/rd/p/000000025.000034252.html

### SUMMARY OF INVENTION

### TECHNICAL PROBLEM

As a result of intensive studies to solve the above problems, the present inventors have discovered that similar culturability to that of conventional Dulbecco's Modified Eagle Medium (DMEM) can be maintained while reducing the addition of amino acids, which are food additives, by replacing a part of the constituents of DMEM with a yeast extract and further adding cystine and/or cysteine, as well as glutamine, and have thus conceived the present invention.

### SOLUTION TO PROBLEM

The present invention includes, but is not limited to, the following aspects:
I
   [1] A basal medium composition for animal cells, containing a yeast extract, amino acids, an inorganic salt and a sugar, wherein the amino acids in the composition include cystine and/or cysteine, and glutamine.
   [2] The basal medium composition for animal cells according to [1], wherein the content of all amino acids in the composition is 0.002 to 1.0 parts by mass per 100 parts by mass of the basal medium composition.
   [3] The basal medium composition for animal cells according to [1] or [2], wherein the percentage of cystine and/or cysteine, and glutamine is 50% or more based on all amino acids in the composition.
   [4] The basal medium composition for animal cells according to any one of [1] to [3], wherein the content of cystine and/or cysteine, and glutamine in the composition is 0.001 to 1.0 parts by mass per 100 parts by mass of the basal medium composition.
   [5] The basal medium composition for animal cells according to any one of [1] to [4], wherein the content of cystine and/or cysteine, and glutamine in the composition is 0.1 to 5.0 parts by mass per solid content of the basal medium composition.
   [6] The basal medium composition for animal cells according to any one of [1] to [5], wherein the content of cystine and/or cysteine in the composition is 0.0001 to 0.02 parts by mass per 100 parts by mass of the basal medium composition.
   [7] The basal medium composition for animal cells according to any one of [1] to [6], wherein the content of cystine and/or cysteine in the composition is 0.005 to 1.0 parts by mass per solid content of the basal medium composition.
   [8] The basal medium composition for animal cells according to any one of [1] to [7], wherein the content of glutamine in the composition is 0.001 to 1.0 parts by mass when the basal medium composition is taken as 100 parts by mass.
   [9] The basal medium composition for animal cells according to any one of [1] to [8], wherein the content of glutamine in the composition is 0.1 to 5.0 parts by mass per solid content of the basal medium composition.
   [10] The basal medium composition for animal cells according to any one of [1] to [9], wherein the yeast extract is an yeast extract with high amino acid content.
   [11] The basal medium composition for animal cells according to any one of [1] to [10], wherein the total amino acid content of the yeast extract is 0.75 parts by mass or more per 100 parts by mass of the basal medium composition.
   [12] The basal medium composition for animal cells according to any one of [1] to [11], wherein the yeast extract is an yeast extract with low nucleic acid content.
   [13] The basal medium composition for animal cells according to any one of [1] to [12], wherein the total nucleic acid content of the yeast extract is 2.0 parts by mass or less per 100 parts by mass of the basal medium composition is taken as 100 parts by mass.
   [14] The basal medium composition for animal cells according to any one of [1] to [13], wherein the yeast extract is an yeast extract with high amino acid content and with low nucleic acid content.
   [15] The basal medium composition for animal cells according to any one of [1] to [14], wherein the content of the yeast extract is 0.05 to 20.0 parts by mass per 100 parts by mass of the basal medium composition is taken as 100 parts by mass.
   [16] The basal medium composition for animal cells according to [1] or [2], further containing a vitamin.
   [17] A method for culturing animal cells, including culturing animal cells in a basal medium for animal cells, wherein the basal medium contains a yeast extract, amino acids, an inorganic salt and a sugar, and the amino acids are cystine and/or cysteine, and glutamine.
   [18] The method according to [17], wherein the yeast extract is an extract from an yeast with high amino acid content, an extract from an yeast with low nucleic acid content, or an extract from an yeast with high amino acid content and with low nucleic acid content.
   [19] An animal cell, which is cultured by the culture method according to [17] or [18].
   [20] A processed food, containing the animal cell according to [19].
II
   [1] A basal medium for animal cells, containing a yeast extract, amino acids, an inorganic salt and a sugar, wherein the amino acids are cystine and glutamine.
   [2] The basal medium for animal cells according to [1], wherein the content of the amino acids is 0.005 to 1.0 parts by mass per 100 parts by mass of the basal medium.
   [3] The basal medium for animal cells according to [1] or [2], wherein the percentage of cystine and glutamine is 50% or more based on the amino acids.
   [4] The basal medium for animal cells according to [1] or [2], wherein the content of cystine and glutamine among the amino acids is 0.0025 to 1.0 parts by mass per 100 parts by mass of the basal medium.
   [5] The basal medium for animal cells according to [1] or [2], wherein the yeast extract is an yeast extract with high amino acid content.
   [6] The basal medium for animal cells according to [1] or [2], wherein the total amino acid content of the yeast extract is 0.75 parts by mass or more per 100 parts by mass of the basal medium.
   [7] The basal medium for animal cells according to [1] or [2], wherein the yeast extract is an yeast extract with low nucleic acid content.
   [8] The basal medium for animal cells according to [1] or [2], wherein the total nucleic acid content of the yeast extract is 2.0 parts by mass or less per 100 parts by mass of the basal medium.
   [9] The basal medium for animal cells according to [1] or [2], wherein the yeast extract is an yeast extract with high amino acid content and with low nucleic acid content.
   [10] The basal medium for animal cells according to [1] or [2], wherein the content of the yeast extract is 0.05 to 20.0 parts by mass per 100 parts by mass of the basal medium.
   [11] The basal medium for animal cells according to [1] or [2], further containing a vitamin.
   [12] The basal medium for animal cells according to [1] or [2], containing calcium chloride as the inorganic salt.
   [13] A method for culturing animal cells, including culturing animal cells in a basal medium for animal cells, wherein the basal medium contains a yeast extract, amino acids, an inorganic salt and a sugar, and the amino acids are cystine and glutamine.
   [14] The method according to [13], wherein the yeast extract is an extract from an yeast with high amino acid content.
   [15] The method according to [13], wherein the yeast extract is an extract from an yeast with low nucleic acid content.
   [16] The method according to [13], wherein the yeast extract is an extract from an yeast with high amino acid content and with low nucleic acid content.
   [17] An animal cell, which is cultured by the culture method according to [13].
   [18] A processed food, containing the animal cell according to [17].
   [19] Use of a yeast extract in combination with amino acids, an inorganic salt and a sugar for a basal medium for animal cells, wherein the amino acids are cystine and glutamine.

### ADVANTAGEOUS EFFECTS OF INVENTION

Yeast extracts are seasonings that contain amino acids, peptides, and nucleic acids found in yeast. By replacing a part of the constituents of a medium with a yeast extract, the present invention reduces the amount of components added to the composition compared to conventional commercially available basal medium compositions, making it possible to reduce the costs required for culturing animal cells and environmental impact.

### BRIEF DESCRIPTION OF DRAWINGS

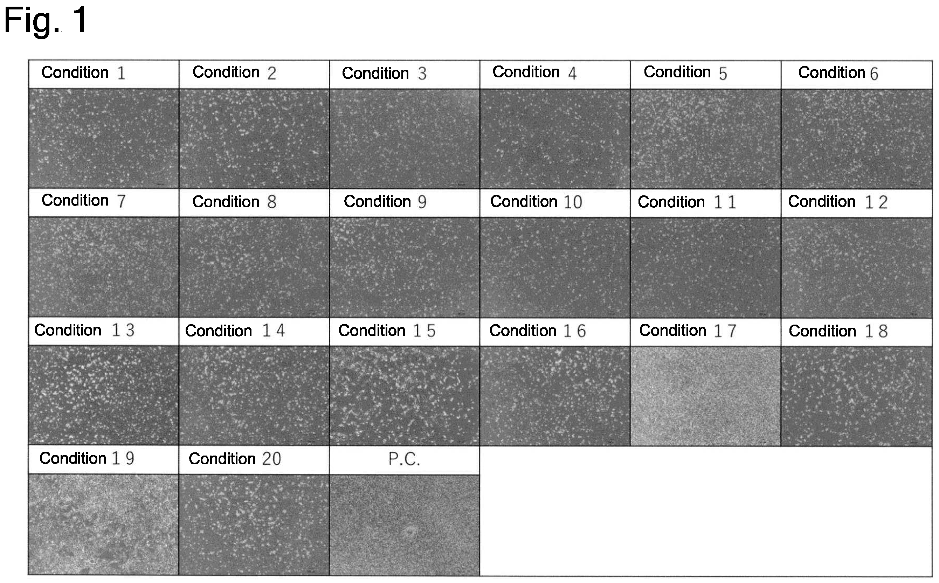

[Fig. 1] Fig. 1 shows the results of cell images in the examination of the minimum amino acid types required to be added in Example 1.

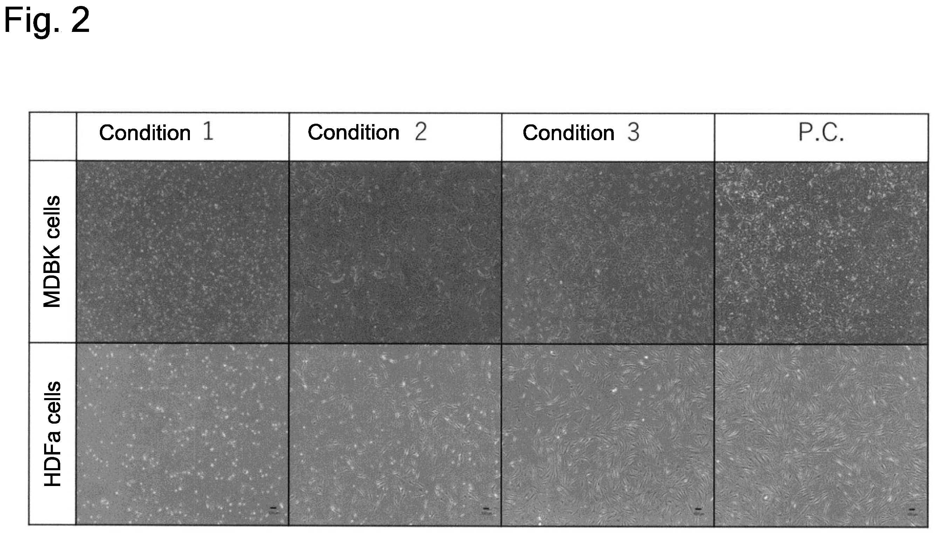

[Fig. 2] Fig. 2 shows the results of cell images in the culture test using MDBK cells and HDFa cells in Example 2.

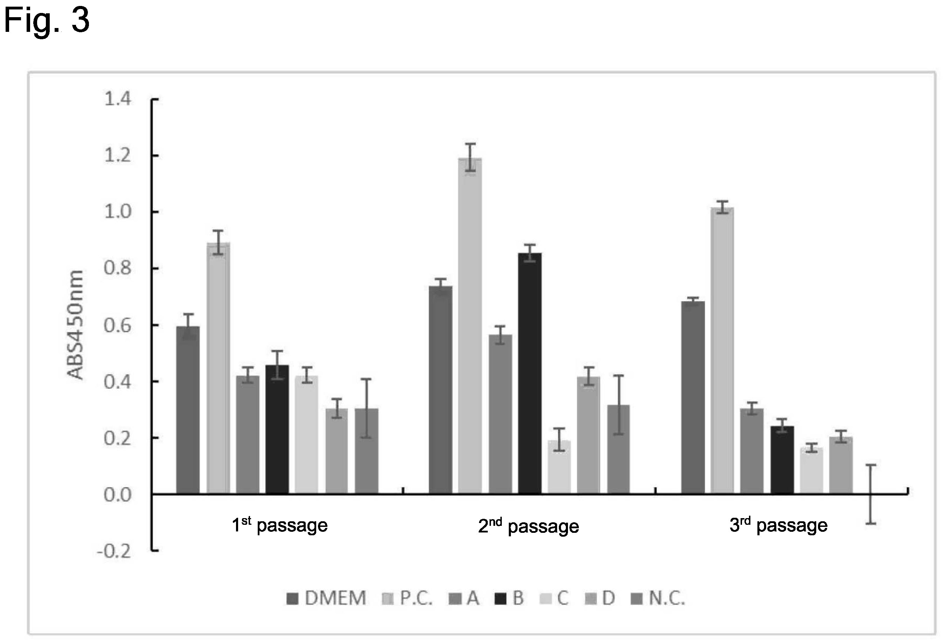

[Fig. 3] Fig. 3 shows the results of comparing the number of viable cells determined by measuring absorbance at 450 nm when various yeast extracts were used in Example 4. The results of each passage are, from the left, those of DMEM, P.C. (positive control), yeast extract A, yeast extract B, yeast extract C, yeast extract D, and N.C. (negative control).

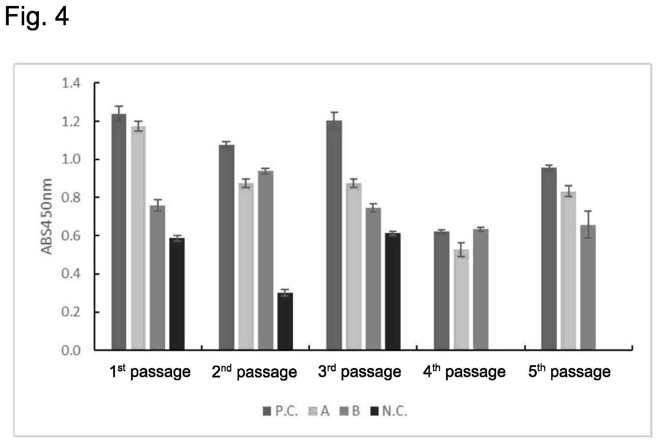

[Fig. 4] Fig. 4 shows the results of comparing the number of viable cells determined by measuring absorbance at 450 nm when yeast extracts A and B were used in Example 5, in which the amount of each yeast extract added was increased to 2.0 g/L and calcium chloride was added to the basal medium. The results of each passage are, from the left, those of P.C. (positive control), yeast extract A, yeast extract B, and N.C. (negative control).

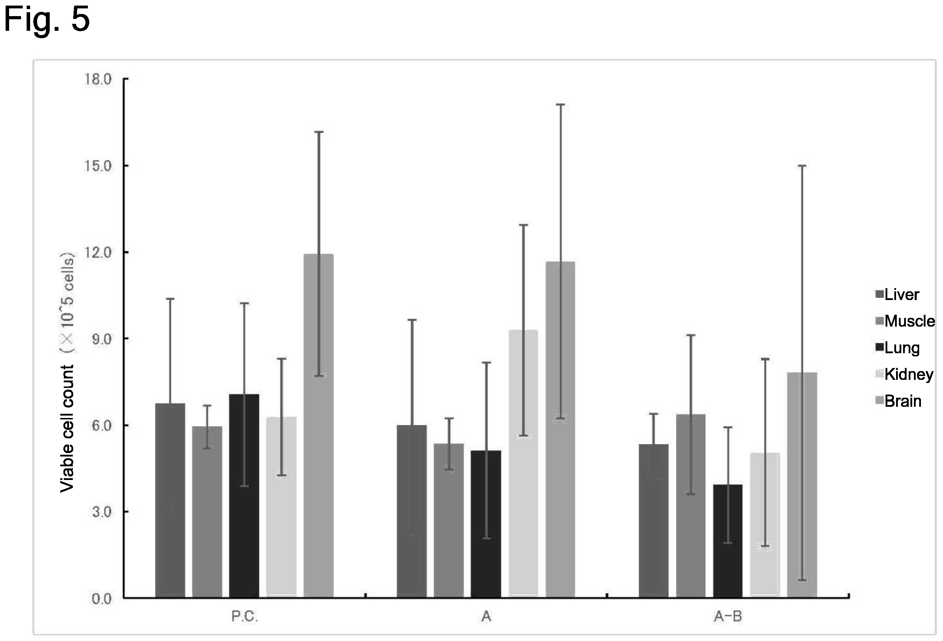

[Fig. 5] Fig. 5 shows the results of measuring the number of viable cells at the 3^{rd} passage when cells derived from various organs extracted from chickens were cultured using yeast extract A and yeast extract A-B in Example 6. The results of each tissue are, from the left, those of liver, muscle, lung, kidney, and brain, in the cases of P.C. (positive control), yeast extract A, and yeast extract A-B.

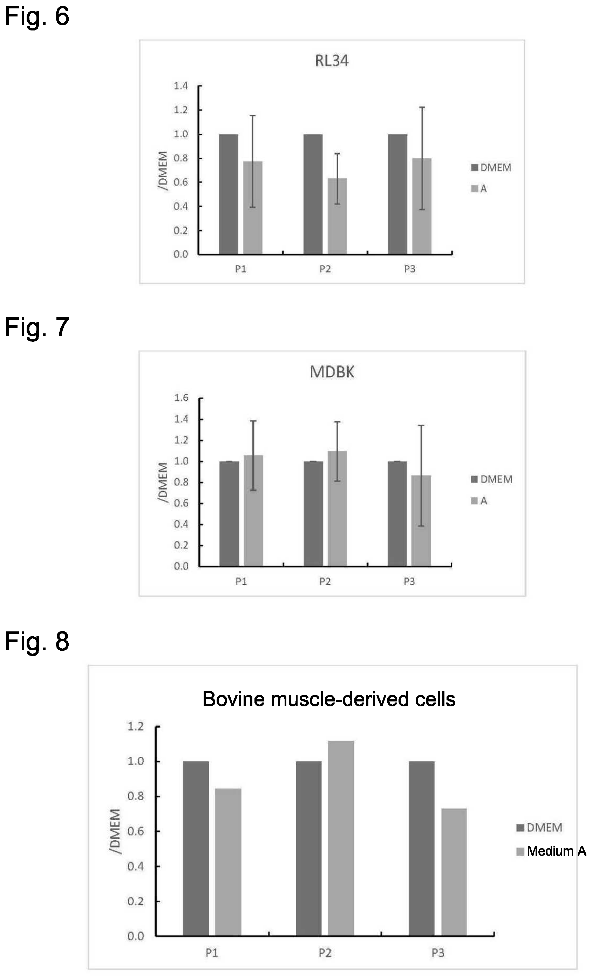

[Fig. 6] Fig. 6 shows the results of examining the number of cells when RL34 cells (cells derived from normal rat liver) were cultured using yeast extract A in Example 7. The vertical axis indicates the value when the number of cells cultured using DMEM as a positive control was defined as 1.0. P1, P2, and P3 on the horizontal axis indicate the results at the 1^{st}, 2^{nd}, and 3^{rd} passages, respectively.
[Fig. 7] Fig. 7 shows the results of examining the number of cells when MDBK cells (cells derived from normal bovine kidney) were cultured using yeast extract A in Example 7. The vertical axis indicates the values when the number of cells cultured using DMEM as a positive control was defined as 1.0. P1, P2, and P3 on the horizontal axis indicate the results at the 1^{st}, 2^{nd}, and 3^{rd} passages, respectively.
[Fig. 8] Fig. 8 shows the results of examining the cell number when bovine muscle-derived cells were cultured using yeast extract A in Example 7. The vertical axis indicates the values when the number of cells cultured using DMEM as a positive control was defined as 1.0. P1, P2, and P3 on the horizontal axis indicate the results at the 1^{st}, 2^{nd}, and 3^{rd} passages, respectively.

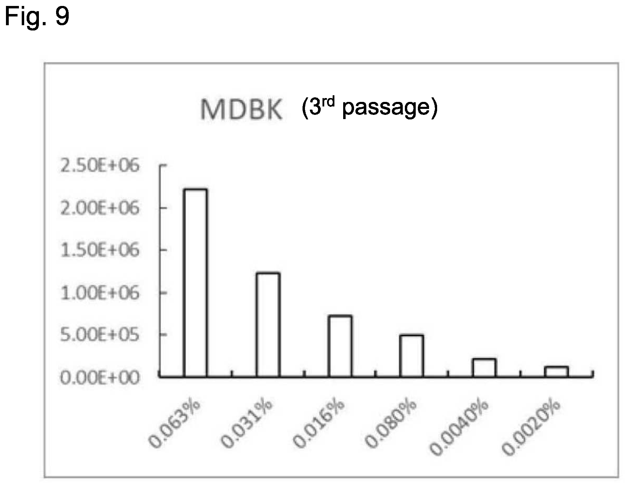

[Fig. 9] Fig. 9 shows the results of subculturing MDBK cells three times using basal media supplemented with various amounts of amino acids in Example 8. The vertical axis of Fig. 9 indicates the number of viable cells.

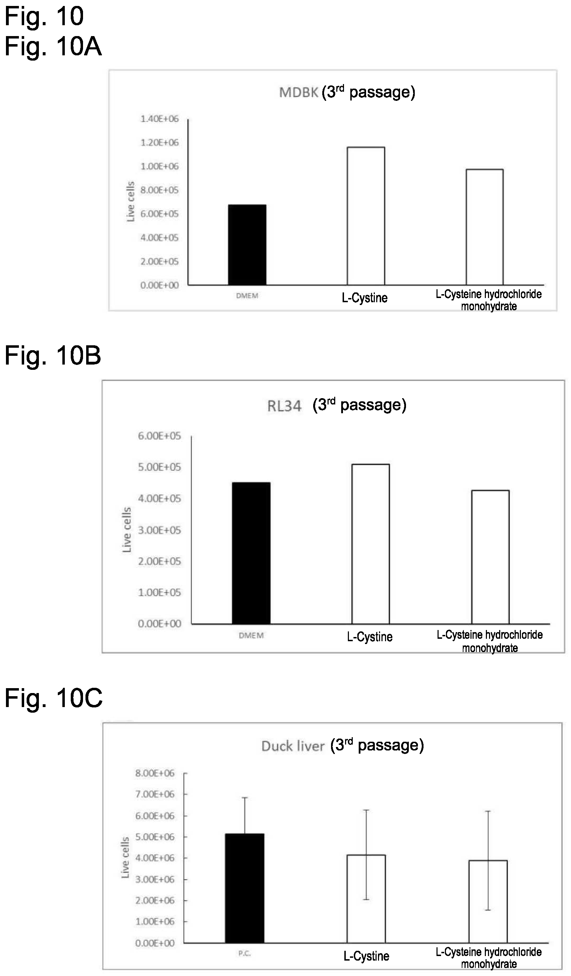

[Fig. 10] Fig. 10 shows the results of subculturing MDBK cells (established cell line) (Fig. 10A), RL34 cells (established cell line) (Fig. 10B), and duck liver-derived cells (primary cultured cells) (Fig. 10C) three times using a basal medium containing L-cysteine or L-cystine. The vertical axis of Fig. 10 indicates the number of viable cells after three times of subculturing.

### DESCRIPTION OF EMBODIMENTS

The present invention includes, but is not limited to, the following aspects. Unless otherwise specified herein, technical and scientific terms used herein have the same meanings as those commonly understood by persons skilled in the art. The substances, materials, and examples disclosed herein are merely illustrative and are not intended to be limiting. When referring to "in one aspect" herein, it means that the invention is not limited the one aspect, i.e., the one aspect is non-limiting.

### 1. Basal medium composition for animal cell culture

In one aspect, the present invention relates to a basal medium composition for animal cells.

The basal medium composition for animal cells contains a yeast extract, amino acids, an inorganic salt and a sugar, and the amino acids in the composition include cystine and/or cysteine, and glutamine. In one aspect, the basal medium composition for animal cells is composed of a yeast extract, amino acids, an inorganic salt and a sugar, and the amino acids are cystine and/or cysteine, and glutamine.

The content of all amino acids in the basal medium composition for animal cells is not particularly limited. The "all amino acids" referred to here are components of the basal medium composition for animal cells, and are added to the basal medium composition for animal cells as individual amino acids, and they are distinguished from the amino acids contained in a yeast extract, which will be described later. "All amino acids" include cystine and/or cysteine as well as glutamine, which are contained as essential components in the composition, and also other types of amino acids added to the basal medium composition for animal cells.

Non-limiting examples of the content of all amino acids in the composition include 5.0 parts by mass or less, 3.0 parts by mass or less, 2.0 parts by mass or less, 1.0 part by mass or less, 0.5 parts by mass or less, 0.1 parts by mass or less, 0.08 parts by mass or less, and 0.07 parts by mass or less, when the basal medium composition is taken as 100 parts by mass. Non-limiting examples of the content of all amino acids in the composition include 0.0005 parts by mass or more, 0.001 parts by mass or more, 0.002 parts by mass or more, 0.003 parts by mass or more, 0.004 parts by mass or more, 0.005 parts by mass or more, 0.01 parts by mass or more, 0.03 parts by mass or more, 0.05 parts by mass or more, and 0.06 parts by mass or more, per 100 parts by mass of the basal medium composition.

Non-limiting examples of the content of all amino acids in the composition include 0.0005 to 5.0 parts by mass, 0.001 to 5.0 parts by mass, 0.001 to 3.0 parts by mass, 0.002 to 3.0 parts by mass, 0.002 to 2.0 parts by mass, 0.002 to 1.0 parts by mass, 0.003 to 2.0 parts by mass, 0.005 to 1.0 parts by mass, 0.01 to 0.5 parts by mass, 0.03 to 0.1 parts by mass, 0.002 parts by mass to 0.07 parts by mass, and 0.004 parts by mass to 0.07 parts by mass when the basal medium composition is taken as 100 parts by mass. Preferably, the amino acid content is 0.002 to 1.0 parts by mass, per 100 parts by mass of the basal medium composition.

The proportion of cystine and/or cysteine and glutamine is not particularly limited. Cystine and/or cysteine and glutamine may be present in equal amounts, or one of them may be present in greater amounts. Preferably, glutamine is present in a greater amount. In one aspect, the amount of glutamine is 1.5 times or more, 2 times or more, 3 times or more, 5 times or more, 8 times or more, or 10 times or more than that of cystine and/or cysteine, in terms of mass. In one aspect, the amount of glutamine is about 12 times that of cystine and/or cysteine.

Cystine, cysteine, and glutamine may be in the L-form or the D-form. Cystine, cysteine, and glutamine may be partially in the L-form, and the remainder in the D-form. In one aspect, cystine, cysteine, and glutamine are in the L-form.

"Amino acids in the composition include cystine and/or cysteine, and glutamine" means that the main amino acid components are cystine and/or cysteine, and glutamine. In one aspect, the percentage of cystine and/or cysteine, and glutamine based on all amino acids in the composition is 50% or more. In one aspect, the percentage of cystine and/or cysteine, and glutamine is 55% or more, 60% or more, 65% or more, 70% or more, 75% or more, 80% or more, 85% or more, 90% or more, 93% or more, 95% or more, 98% or more, or 99% or more, based on all amino acids in the composition. In one aspect, the percentage of cystine and/or cysteine, and glutamine is 100% based on all amino acids in the composition. However, it is not limited to "the percentage of cystine and/or cysteine, and glutamine is 100%," and it is presumed that the nutritional value and therefore proliferation ability may be increased when the larger amounts of other amino acids are added to the composition.

Non-limiting examples of the content of cystine and/or cysteine, and glutamine among the amino acids in the composition include 0.0005 parts by mass or more, 0.0008 parts by mass or more, 0.001 parts by mass or more, 0.002 parts by mass or more, 0.0025 parts by mass or more, 0.003 parts by mass or more, 0.005 parts by mass or more, 0.01 parts by mass or more, 0.03 parts by mass or more, 0.05 parts by mass or more, and 0.06 parts by mass or more, per 100 parts by mass of the basal medium composition.

Non-limiting examples of the content of cystine and/or cysteine, and glutamine among the amino acids in the composition include 0.00025 to 5.0 parts by mass, 0.0005 to 5.0 parts by mass, 0.0008 parts by mass to 5.0 parts by mass, 0.0008 parts by mass to 3.0 parts by mass, 0.001 to 5.0 parts by mass, 0.001 to 3.0 parts by mass, 0.001 to 2.0 parts by mass, 0.001 to 1.0 parts by mass, 0.002 to 5.0 parts by mass, 0.002 to 3.0 parts by mass, 0.002 to 1.0 parts by mass, 0.0025 to 1.0 parts by mass, 0.003 to 2.0 parts by mass, 0.005 to 1.0 parts by mass, 0.01 to 0.5 parts by mass, 0.03 to 0.1 parts by mass, and 0.002 parts by mass to 0.07 parts by mass, per 100 parts by mass of the basal medium composition. In one aspect, the content of cystine and/or cysteine, and glutamine among the amino acids in the composition is 0.001 to 1.0 parts by mass, per 100 parts by mass of the basal medium composition. In one aspect, the content of cystine and/or cysteine, and glutamine among the amino acids in the composition is 0.002 to 1.0 parts by mass per 100 parts by mass of the basal medium composition.

Non-limiting examples of the content of cystine and/or cysteine, and glutamine in the composition include 0.025 to 25.0 parts by mass, 0.05 to 25.0 parts by mass, 0.08 parts by mass to 25.0 parts by mass, 0.08 parts by mass to 15.0 parts by mass, 0.1 to 25.0 parts by mass, 0.1 to 15.0 parts by mass, 0.1 to 10.0 parts by mass, 0.1 to 5.0 parts by mass, 0.2 to 25.0 parts by mass, 0.2 to 15 parts by mass, 0.2 to 5.0 parts by mass, 0.25 to 5.0 parts by mass, 0.3 to 10.0 parts by mass, 0.5 to 5.0 parts by mass, 0.3 to 2.5 parts by mass, 0.4 to 2.0 parts by mass, and 0.1 to 3.5 parts by mass, per solid content of the basal medium composition. In one aspect, the content of cystine and/or cysteine, and glutamine in the composition is 0.1 to 5.0 parts by mass, per solid content of the basal medium composition. In one aspect, the content of cystine and/or cysteine, and glutamine in the composition is 0.2 to 5.0 parts by mass, per solid content of the basal medium composition.

Non-limiting examples of the content of cystine and/or cysteine in the composition include 0.00001 to 0.2 parts by mass, 0.00005 to 0.15 parts by mass, 0.00008 to 0.1 parts by mass, 0.0001 to 0.1 parts by mass, 0.0001 to 0.08 parts by mass, 0.0001 to 0.04 parts by mass, 0.0001 to 0.02 parts by mass, 0.0001 to 0.01 parts by mass, 0.0002 to 0.1 parts by mass, 0.0002 to 0.08 parts by mass, 0.0002 to 0.05 parts by mass, 0.0002 to 0.02 parts by mass, 0.0002 to 0.01 parts by mass, 0.0002 to 0.005 parts by mass, and 0.0004 to 0.008 parts by mass, per 100 parts by mass of the basal medium composition. In one aspect, the content of cystine and/or cysteine in the composition is 0.0001 to 0.02 parts by mass per 100 parts by mass of the basal medium composition.

Non-limiting examples of the content of cystine and/or cysteine in the composition include 0.0005 to 10.0 parts by mass, 0.0025 to 7.5 parts by mass, 0.004 to 5.0 parts by mass, 0.005 to 5.0 parts by mass, 0.005 to 4.0 parts by mass, 0.005 to 2.0 parts by mass, 0.005 to 1.0 parts by mass, 0.005 to 0.5 parts by mass, 0.01 to 5.0 parts by mass, 0.01 to 4.0 parts by mass, 0.01 to 2.5 parts by mass, 0.01 to 1.0 parts by mass, 0.01 to 0.5 parts by mass, 0.01 to 0.30 parts by mass, and 0.02 to 0.40 parts by mass, per solid content of the basal medium composition. In one aspect, the content of cystine and/or cysteine in the composition is 0.005 to 1.0 parts by mass per solid content of the basal medium composition.

Non-limiting examples of the content of glutamine among the amino acids in the composition include 0.00025 to 5.0 parts by mass, 0.0005 to 5.0 parts by mass, 0.0008 parts by mass to 5.0 parts by mass, 0.0008 parts by mass to 3.0 parts by mass, 0.001 to 5.0 parts by mass, 0.001 to 3.0 parts by mass, 0.001 to 2.0 parts by mass, 0.001 to 1.0 parts by mass, 0.002 to 5.0 parts by mass, 0.002 to 3.0 parts by mass, 0.002 to 1.0 parts by mass, 0.0025 to 1.0 parts by mass, 0.003 to 2.0 parts by mass, 0.005 to 1.0 parts by mass, 0.01 to 0.5 parts by mass, 0.03 to 0.1 parts by mass, and 0.002 parts by mass to 0.06 parts by mass, per 100 parts by mass of the basal medium composition. In one aspect, the content of glutamine among the amino acids in the composition is 0.001 to 1.0 parts by mass per 100 parts by mass of the basal medium composition. In one aspect, the content of glutamine among the amino acids in the composition is 0.002 to 1.0 parts by mass when per 100 parts by mass of the basal medium.

Non-limiting examples of the content of glutamine in the composition include 0.025 to 25.0 parts by mass, 0.05 to 25.0 parts by mass, 0.08 parts by mass to 25.0 parts by mass, 0.08 parts by mass to 15.0 parts by mass, 0.1 to 25.0 parts by mass, 0.1 to 15.0 parts by mass, 0.1 to 10.0 parts by mass, 0.1 to 5.0 parts by mass, 0.2 to 25.0 parts by mass, 0.2 to 15 parts by mass, 0.2 to 5.0 parts by mass, 0.25 to 5.0 parts by mass, 0.3 to 10.0 parts by mass, 0.5 to 5.0 parts by mass, 0.3 to 2.5 parts by mass, 0.4 to 2.0 parts by mass, and 0.1 to 3.2 parts by mass, per solid content of the basal medium composition. In one aspect, the content of glutamine in the composition is 0.1 to 5.0 parts by mass per solid content of the basal medium composition. In one aspect, the content of glutamine in the composition is 0.2 to 5.0 parts by mass per solid content of the basal medium composition.

The amino acids contained in the basal medium composition for animal cells are cystine and/or cysteine, and glutamine, and preferably do not include amino acids other than cystine and/or cysteine, and glutamine. The "amino acids other than cystine and/or cysteine, and glutamine" are not particularly limited and include natural amino acids and non-natural amino acids. In one aspect, the amino acids other than cystine and/or cysteine, and glutamine include isoleucine, leucine, threonine, tryptophan, valine, histidine, phenylalanine, methionine, lysine (including lysine hydrochloride), tyrosine, arginine, glycine, and serine.

"Yeast extract" (also referred to as "yeast extract") is a substance obtained by extraction of components contained in yeast fungal bodies therefrom. In general, the main components are proteins and peptides degraded from proteins, amino acid and nucleic acid-related substances, minerals, and vitamins, and the use of such a yeast extract as a seasoning, livestock feed, dietary supplement, medium for culturing microorganisms, and quality improver for processed foods, and the like is known.

Amino acids contained in a yeast extract are components of the basal medium composition for animal cells, and are distinct from the amino acids that are added as individual amino acids to the basal medium composition for animal cells (i.e., including cystine and/or cysteine, and glutamine).

The type of yeast cells from which the yeast extract contained in the basal medium composition for animal cells is derived is not particularly limited. In one aspect (i.e., non-limiting), examples of yeast cells include baker's yeast, brewer's yeast, sake yeast, and torula yeast cells. In one aspect, baker's yeast (scientific name: *Saccharomyces cerevisiae*) is included.

Known examples of the yeast extract include yeast extracts with high amino acid content that contain a large amount of amino acids, yeast extracts with high nucleic acid content and with high with high amino acid content, and yeast extracts with high nucleic acid contents, according to the components contained. Yeast extracts that contain a large amount of specific amino acids, organic acids, and/or nucleic acids are also known. Particular examples thereof include: glutamic acid yeast extracts with high amino acid content that contain glutamic acid in a high concentration, particularly among amino acids; yeast extracts with high succinic acid content that contain succinic acid in a high concentration among organic acids; and yeast extracts with high nucleic acid content that contain inosinic acid and guanylic acid in high concentrations, among nucleic acids.

Without limitation, the yeast extract is an yeast extract with high amino acid content . Without limitation, the yeast extract is an yeast extract with low nucleic acid content. Without limitation, the yeast extract is an yeast extract with high amino acid content and with low nucleic acid content.

In one aspect, the yeast extract with high amino acid content contains 30,000 mg or more, 35,000 mg or more, 40,000 mg or more, or 45,000 mg or more of total amino acids, per 100 g of the yeast extract.

When 100 g of the yeast extract contains 30,000 mg of total amino acids, the amino acid content in the yeast extract is 30%. In Example 3 described below, the yeast extract was added so that the final concentration in a culture medium was 0.25 g/L to 2.0 g/L. When the amino acid content in the yeast extract is 30%, the amino acid concentration in the culture medium is 0.075 g/L to 0.6 g/L (1,000 g). In this case, the total amino acid content in the yeast extract is 0.75 parts by mass to 6.0 parts by mass per 100 parts by mass of the basal medium composition.

Non-limiting examples of the total amino acid content of the yeast extract include 0.30 parts by mass or more, 0.50 parts by mass or more, 0.60 parts by mass or more, 0.75 parts by mass or more, 1.0 part by mass or more, or 1.5 parts by mass or more, per 100 parts by mass of the basal medium composition. In one aspect, the total amino acid content of the yeast extract is 0.75 parts by mass or more, per 100 parts by mass of the basal medium composition.

Non-limiting examples of the total amino acid content of the yeast extract include 20 parts by mass or less, 15 parts by mass or less, 10 parts by mass or less, 8 parts by mass or less, or 6 parts by mass or less, per 100 parts by mass of the basal medium composition.

Non-limiting examples of the total amino acid content of the yeast extract include 0.30 parts by mass or more and 20 parts by mass or less, 0.50 parts by mass or more and 10 parts by mass or less, 0.60 parts by mass or more and 8 parts by mass or less, and 0.75 parts by mass or more and 6 parts by mass or less, per 100 parts by mass of the basal medium composition.

In one aspect, the yeast extract with high nucleic acid content contains 10% or less, 8% or less, 5% or less, 3% or less, or 2% or less nucleic acids based on the yeast extract.

In Example 3 described below, the yeast extract was added so that the final concentration in the culture medium was 0.25 g/L to 2.0 g/L. When the nucleic acid content in the yeast extract is 10%, the nucleic acid concentration of the culture medium is 0.025 g/L to 0.2 g/L (1,000 g). In this case, the total nucleic acid content of the yeast extract is 0.25 parts by mass to 2.0 parts by mass per 100 parts by mass of the basal medium composition is taken as 100 parts by mass.

Non-limiting examples of the total nucleic acid content of the yeast extract include 3.0 parts by mass or less, 2.0 parts by mass or less, 1.5 parts by mass or less, 1.0 part by mass or less, 0.7 parts by mass or less, 0.5 parts by mass or less, and 0.3 parts by mass or less, per 100 parts by mass of the basal medium composition. In one aspect, the total nucleic acid content of the yeast extract is 2.0 parts by mass or less, per 100 parts by mass of the basal medium composition.

Non-limiting examples of the total nucleic acid content of the yeast extract include 0.01 parts by mass or more, 0.05 parts by mass or more, 0.10 parts by mass or more, 0.20 parts by mass or more, and 0.25 parts by mass or more, per 100 parts by mass of the basal medium composition. In one aspect, the total nucleic acid content of the yeast extract is 0.25 parts by mass or more, per 100 parts by mass of the basal medium composition.

Non-limiting examples of the total nucleic acid content of the yeast extract include 0.05 parts by mass or more to 2.0 parts by mass or less, 0.10 parts by mass or more to 2.0 parts by mass or less, 0.20 parts by mass or more to 2.0 parts by mass or less, and 0.25 parts by mass or more and 2.0 parts by mass or less, per 100 parts by mass of the basal medium composition.

The yeast extract contained in the basal medium composition for animal culture may be one type, or two or more types.

Without limitation, the yeast extract may be any one of yeast extracts A, B, C, and D described in the Examples below, or a mixture thereof. In one aspect, the yeast extract may also be yeast extract A or B described in the Examples below, or a mixture thereof. In one aspect, the yeast extract may be yeast extract A described in the Examples below. Specific examples thereof include yeast extract YK-21NL, Himax PR, YP21-CM, and yeast extract 21-NYP (all sold by Fuji Foods Corporation).

The content of the yeast extract in the basal medium composition for animal cells is not particularly limited. The content of the yeast extract is desirably a concentration such that the effect of proliferating animal cells can be obtained without inhibition of proliferation (due to too high concentration). Non-limiting examples of the content of the yeast extract in the basal medium composition for animal cells include 0.01 to 25.0 parts by mass, 0.05 to 20.0 parts by mass, 0.10 to 15.0 parts by mass, 0.20 to 10.0 parts by mass, 0.25 to 5.0 parts by mass, and 0.25 g to 2.0 parts by mass, per 100 parts by mass of the basal medium composition. In one aspect, the content of the yeast extract in the basal medium composition for animal cells is 0.05 to 20.0 parts by mass, per 100 parts by mass of the basal medium composition.

Combined uses with the yeast extract, or those other than the yeast extract, extracts derived from natural products other than the yeast extract may be used as components of the basal medium composition for animal cells. Non limiting examples of the extract derived from a natural product can include extracts derived from: grains such as corn, wheat, barley, and rice; beans (excluding soybeans) such as kidney beans and peas; animal tissues such as meat and bones; or microbial tissues such as from fungal bodies and mycelium.

In one aspect, examples of the extract derived from a natural product other than the yeast extract does not include soybean hydrolysate.

The type of the inorganic salt contained in the basal medium composition for animal cells is not particularly limited. Non-limiting examples thereof include salts of calcium, iron, potassium, magnesium, sodium, and zinc. Non-limiting examples of salts include chlorides, sulfates, phosphates, hydrochlorides, bicarbonates, and nitrates. Non-limiting examples of the inorganic salt include calcium chloride, iron chloride, potassium chloride, magnesium sulfate, sodium chloride, sodium dihydrogen phosphate, sodium bicarbonate, ferrous sulfate, ferrous nitrate, magnesium chloride, and zinc sulfate. In one aspect, calcium chloride is contained as the inorganic salt. In one aspect, the basal medium composition for animal cells may contain two or more types of inorganic salts.

In one aspect, the basal medium composition for animal cells contains two or more types of inorganic salts selected from the group consisting of calcium chloride, potassium chloride, sodium chloride, and sodium bicarbonate. In one aspect, the basal medium composition for animal cells contains calcium chloride, potassium chloride, sodium chloride, and sodium bicarbonate.

The amount of the inorganic salt contained in the basal medium composition for animal cells is not particularly limited. Depending on the type of inorganic salt, a concentration appropriate for culturing animal cells can be appropriately adopted.

"Sugar" is the first oxidation product of polyhydric alcohol and has one formyl group (-CHO) or one carbonyl group (>C=O). Sugars with a formyl group are classified as aldoses, and sugars with a carbonyl group are classified as ketoses. Sugar is abundant in plant tissues, honey, and fruits, and in particular glucose is a source of energy for living organisms.

When two monosaccharide molecules are joined together by a glycosidic bond to be one molecule, the resultant is called disaccharide. When three monosaccharide molecules are joined together, the resultant is called trisaccharide, and similarly when four molecules are joined together, the resultant is called tetrasaccharide. When about 2 to 20 monosaccharide molecules are joined together, the resultant is called oligosaccharide. When many monosaccharides are polymerized through glycosidic bonds, the resultant is called polysaccharide. When the hydroxy group of a sugar is replaced with hydrogen, the resultant is called deoxysugar. When the terminal carbon of an aldose is replaced with a carboxyl group, the resultant is called uronic acid. When the hydroxy group is replaced with an amino group, the resultant is called aminosugar. When a ketone or aldehyde group is reduced to an alcohol, the resultant is called sugar alcohol.

Known examples of sugars include monosaccharide such as glucose (grape sugar), mannose, fructose (fruit sugar), galactose, and xylose. Known examples of disaccharides consisting of two glucose molecules include trehalose, isotrehalose, maltose (malt sugar), cellobiose, and isomaltose. Known examples of other disaccharides include lactose (milk sugar) (1,4-galactosidic bond between β-galactose and β-glucose) and sucrose (cane sugar) (1,2-glycosidic bond between α-glucose and β-fructofuranose (fructose)).

The type of sugar contained in the basal medium composition for animal cells is not particularly limited. Non-limiting examples thereof include glucose (grape sugar), fructose, trehalose, galactose, sucrose, and maltose. In one aspect, the sugar contained in the basal medium composition for animal cells is glucose (grape sugar). In one aspect, the basal medium composition for animal cells may contain two or more types of sugar.

The amount of the sugar contained in the basal medium composition for animal cells is not particularly limited. Depending on the type of sugar, a concentration appropriate for culturing animal cells can be appropriately adopted.

In one aspect, the basal medium composition for animal cells may further contain a vitamin(s).

Of nutrients necessary in small amounts for the survival and growth of living organisms, "vitamin" is a general term for organic compounds other than carbohydrates, proteins, and lipids that cannot be synthesized in sufficient quantities within the body of the organism. Vitamins are classified by function, not by substance name. For example, vitamin A includes retinal, retinol, and the like. Vitamins are classified into fat-soluble and water-soluble vitamins. The following are examples of vitamins for humans.

### Fat-soluble vitamins

Vitamin A: Retinol, β-carotene, α-carotene, β-cryptoxanthin, etc.
Vitamin D: Ergocalciferol, Cholecalciferol
Vitamin E: Tocopherol, Tocotrienol
Vitamin K: Two naphthoquinone derivatives, phylloquinone and menaquinone Water-soluble vitamins

### Vitamin B group

Vitamin B₁: Thiamine
Vitamin B₂: Riboflavin, also known as vitamin G.
Vitamin B₃: Niacin, also known as vitamin PP
Vitamin B₅: Pantothenic acid
Vitamin B₆: Pyridoxal, Pyridoxamine, Pyridoxine
Vitamin B₇: Biotin, also known as vitamin Bw and vitamin H.
Vitamin B₉: Folic acid, also known as vitamin Bc and vitamin M.
Vitamin B₁₂: Cyanocobalamin, Methylcobalamin, Hydroxocobalamin

### Vitamin C: Ascorbic acid

Substances that do not fit the definition of vitamins above but have effects similar to those of vitamins are sometimes referred to as "vitamin-like substances." Vitamin-like substances include those that were historically mistakenly thought to be vitamins, or those that are no longer considered vitamins due to changes in the definition. The following are examples of vitamin-like substances.

### Vitamin B₄: Adenine

Vitamin B₈: Ergadenylic acid (adenylic acid)
Vitamin B₁₀: A mixture of various B vitamins including folic acid. Also known as vitamin R.
Vitamin B₁₁: Folic acid analogue.
Vitamin B₁₃: Orotic acid
Vitamin B₁₄: A mixture of folic acid or lipoic acid, etc.
Vitamin B₁₅: Pangamic acid
Vitamin B₁₆: Dimethylglycine
Vitamin B₁₇: Amygdalin
Vitamin B_{H}: Inositol
Vitamin B_{P}: Choline
Vitamin B_{T}: Carnitine
Vitamin B_{X}: para-Aminobenzoic acid (partial structure of folic acid, also known as PABA)
Vitamin F: Essential fatty acids such as linoleic acid
Vitamin I: Rice bran extract.
Vitamin J: Catechol, Flavin or Choline
Vitamin L₁: Anthranilic acid
Vitamin L₂: Adenylthiomethylpentose
Vitamin N: Thioctic acid (α-Lipoic acid)
Vitamin O: Carnitine
Vitamin P: Flavonoids such as quercetin, hesperidin, rutin, and eriocitrin
Vitamin Q: Ubiquinone
Vitamin S: Salicylic acid
Vitamin T: Tegotin
Vitamin U: Methylmethionine sulfonium chloride (also referred to as "Cabagin")
Vitamin V: Nicotinamide adenine dinucleotide

The type of vitamin contained in the basal medium composition for animal cells is not particularly limited. As used herein, "vitamins" include the above vitamin-like substances, unless otherwise specified.

Without limitation, the vitamin(s) contained in the basal medium composition for animal cells is one or two or more vitamins selected from the group consisting of pantothenate (e.g., calcium pantothenate), folic acid, pyridoxine or a salt thereof (e.g., pyridoxyl hydrochloride), riboflavin, thiamine or a salt thereof (e.g., thiamine hydrochloride), choline (e.g., glycerophosphatidylcholine), inositol (e.g., myo-inositol), and nicotinamide (vitamin V). In one aspect, the basal medium composition for animal cells contains all of pantothenate (e.g., calcium pantothenate), folic acid, pyridoxine or a salt thereof (e.g., pyridoxyl hydrochloride), riboflavin, thiamine or a salt thereof (e.g., thiamine hydrochloride), choline (e.g., glycerophosphatidylcholine), inositol (e.g., myo-inositol), and nicotinamide (vitamin V).

The amount of the vitamin contained in the basal medium composition for animal cells is not particularly limited. Depending on the type of vitamin, a concentration appropriate for culturing animal cells can be appropriately adopted.

The basal medium composition for animal cells may contain other components in addition to yeast extracts, amino acids, inorganic salts, vitamins and sugars. The other components may be any components that can be used in the basal medium composition for animal cells, and examples thereof include pH indicators (phenol red), glutathione, fatty acids such as linolenic acid, and thymidine.

The "basal medium for animal cells" refers to a medium that is the base of a culture medium for culturing, proliferating, and/or differentiating animal cells, and is used non-limitingly usually with the addition of serum or the like as appropriate. As used herein, "culture" is used to mean proliferation, unless otherwise specified. As used herein, "basal medium composition for animal cells" refers to a composition that is the base of a basal medium for animal cells for culturing animal cells. The basal medium composition for animal cells may be a liquid composition or a solid composition. The basal medium composition for animal cells may be, for example, a liquid composition and may be directly used as a basal medium for animal cells. It may also be in the form of a paste from which water has been removed, may be stored, sold, etc., by adjusting it to have appropriate water activity, and may be used as a basal medium for animal cells by adding an appropriate amount of water. Alternatively, it may be a solid composition that is stored and/or sold, etc., in the form of a powdered material that has been dried, etc., and may be dissolved in a solvent (e.g., water) when used and used as a basal medium for animal cells. As used herein, "basal medium for animal cells" or "basal medium" may be used synonymously with "basal medium composition for animal cells".

The types of animals, from which animal cells are derived, are not particularly limited. In one aspect, the animal cells are cells of a vertebrate such as a mammal. The vertebrate may be any of mammals, reptiles, birds, and amphibians, and examples thereof include humans, cows, pigs, rabbits, chickens, sheep, and goats. In one aspect, the vertebrate is a mammal other than a human. In one aspect, the animal cells are cells derived from (oviparous) animals that lay eggs with shells, such as birds and reptiles. In one aspect, the animal cells are chicken cells.

The types of animal cells are not particularly limited, and may be any of, for example, somatic cells derived from adults, embryos, cultured cells established from fertilized eggs, etc. Without limitation, for example, in the case of chickens, chicken embryos can be used, and preferably 0-day to 21-day embryos, 2-day to 19-day embryos, 4-day to 17-day embryos, and 6-day to 15-day embryos can be used.

The tissues from which the animal cells are derived are not particularly limited. Non-limiting examples of the animal cells include muscle-derived cells, skin-derived cells, intestine-derived cells, heart-derived cells, brain-derived cells, stomach-derived cells, embryonic membrane-derived cells, liver-derived cells, kidney-derived cells, and lung-derived cells.

In the following aspect, amino acids are cystine and/or cysteine, and glutamine, and the amino acids that are cystine and/or cysteine, and glutamine are the main components. In one aspect, amino acids other than cystine and/or cysteine, and glutamine are not contained, but are not limited thereto. Although cystine and/or cysteine, and glutamine are essential elements of the basal medium composition for animal cells, it is presumed that the growth of the animal cells may be promoted nutritionally by combining them with other amino acids.

In one aspect, the present invention is the use of a yeast extract in combination with amino acids, an inorganic salt and a sugar for the basal medium composition for animal cells, wherein the amino acids are cystine and/or cysteine, and glutamine.

In one aspect, the present invention relates to a combination of a yeast extract, amino acids, an inorganic salt and a sugar for use in the basal medium composition for animal cells, wherein the amino acids are cystine and/or cysteine, and glutamine.

In one aspect, the present invention relates to a kit for producing the basal medium composition for animal cells, containing a yeast extract, amino acids, an inorganic salt, and a sugar, wherein the amino acids are cystine and/or cysteine, and glutamine. In the kit, each of the components including a yeast extract, amino acids, an inorganic salt, and a sugar, may exist in an individual state, or two or more of the components may exist in a mixed state until the basal medium composition for animal cells is produced.

### 2. Method for culturing animal cells

In one aspect, the present invention relates to a method for culturing animal cells.

Without limitation, the culture method includes culturing animal cells in a basal medium for animal cells, wherein the basal medium contains a yeast extract, amino acids, an inorganic salt, and a sugar, and the amino acids include L-cystine and/or L-cysteine, and L-glutamine.

The culture conditions in the above method for culturing animal cells are not particularly limited. Appropriate culture conditions can be adopted depending on the animal species, tissue, and the like from which the animal cells are derived.

In one aspect, the yeast extract is an extract from an yeast with high amino acid content.

In one aspect, the yeast extract is an extract from an yeast with low nucleic acid-content.

In one aspect, the yeast extract is an extract from an yeast with high amino acid content and with low nucleic acid content.

In one aspect, the basal medium for animal cells used in the method for culturing animal cells may contain a vitamin.

The meanings of "yeast extract," "amino acids," "inorganic salt," "vitamin," "sugar," and "basal medium for animal cells" are as described in "1. Basal medium composition for animal cell culture."

The matters described in "1. Basal medium composition for animal cell culture" are also applicable to the culture method of this section unless otherwise inconsistent.

In one aspect, the present invention relates to a medium for a method for culturing animal cells, wherein the medium contains a yeast extract, amino acids, an inorganic salt and a sugar, and the amino acids include L-cystine and/or L-cysteine, and L-glutamine.

In one aspect, the present invention relates to the use of a medium for the method for culturing animal cells, wherein the medium contains a yeast extract, amino acids, an inorganic salt and a sugar, and the amino acids include L-cystine and/or L-cysteine, and L-glutamine.

### 3. Animal cells and processed products

In one aspect, the present invention includes animal cells cultured by the method for culturing animal cells of the present invention.

In one aspect, the present invention relates to a processed food containing the animal cells.

The meanings of "method for culturing animal cells", "animal cells" and others are as described in "1. Basal medium composition for animal cell culture" and "2. Method for culturing animal cells".

The matters described in "1. Basal medium composition for animal cell culture" and "2. Method for culturing animal cells" are also applicable to the animal cells and processed foods in this section, unless otherwise inconsistent.

Animal cells cultured by the method for culturing animal cells of the present invention and processed foods can be produced with less environmental impact than those produced in cases where conventional commercially available basal medium compositions are used. As a result, animal cells and processed foods can be obtained at low cost and with less environmental impact. In addition, they also have a more complex flavor that could not be obtained by methods for culturing animal cells, and also have the characteristic of having the unique meat flavor that is inherent to animal tissue.

Examples of "processed foods" include meat (cultured meat) that contains cultured animal cells or is made from cultured animal cells. Cultured meat can be cooked and used as a food material in the same way as regular meat such as meat from cows, pigs, rabbits, chickens, sheep, goats, and others. The "food" in "processed foods" can mean either food materials before cooking or foods (meals) after cooking. The "process" in "processed foods" not only simply means cooking food materials to create a meal, but also means carrying out generally processing of food materials, such as preliminarily processing food materials to produce more highly processed food materials, or processing food materials to provide new food materials with modified tastes or physical properties, for example.

### EXAMPLES

The present invention will be described in detail below by way of examples, but the present invention is not limited to these examples. Persons skilled in the art can easily modify and alter the present invention based on the description given herein, and such modifications and alterations are within the technical scope of the present invention.

### Example 1 Examination of the minimum amino acid types required to be added

In this example, examination was conducted to determine whether or not animal cells can proliferate using a yeast extract alone without the addition of additional amino acids when culturing the animal cells using the yeast extract as an amino acid source, and if not, which amino acids are the minimum amino acids required for cell proliferation.

### (Method)

Of the constituents to be contained in Dulbecco's Modified Eagle Medium (DMEM), those excluding amino acids was dissolved in ion-exchanged water to prepare A.A.-IMEM Buffer (Table 1).

**Table 1 A.A.-IMEM Buffer formulation**

| [Table 1] | | |
|---|---|---|
| Substance name | mg/L | Reagent manufacturer |
| Calcium chloride (granular) | 378 | KANTO CHEMICAL CO., INC. |
| Iron (III) chloride hexahydrate | 0.068 | Junsei Chemical Co., Ltd. |
| Potassium chloride | 404 | KANTO CHEMICAL CO., INC. |
| Magnesium sulfate (crystal) | 202 | KANTO CHEMICAL CO., INC. |
| Common salt | 5726 | Commercial product |
| Sodium dihydrogen phosphate (crystal) | 144 | KANTO CHEMICAL CO., INC. |
| Calcium pantothenate | 4 | Kongo Yakuhin Co., Ltd. |
| Glycero phosphatidylcholine | 8 | Bio Actives Japan Corporation |
| Folic acid | 4 | Kongo Yakuhin Co., Ltd. |
| myo-Inositol | 7 | TSUNO CO., LTD. |
| Nicotinamide | 4 | Kongo Yakuhin Co., Ltd. |
| Pyridoxyl hydrochloride | 4 | Kongo Yakuhin Co., Ltd. |
| Riboflavin | 0.4 | Kongo Yakuhin Co., Ltd. |
| Thiamine hydrochloride | 4 | Kongo Yakuhin Co., Ltd. |
| Grape sugar | 5000 | Kanematsu Ensho Co., Ltd. |
| Sodium bicarbonate | 3737 | KANTO CHEMICAL CO., INC. |

Seven types of yeast extract (B, D, E, F, H, I, and J) shown below were each further added to the A.A.-IMEM Buffer, so that the final concentration in the medium was 1 g/L.

**Table 2 Yeast extracts added in Example 1**

| [Table 2] | | | |
|---|---|---|---|
| | Advantage of each yeast extract | Total amount of amino acids (mg/100g) | Total amount of nucleic acids (%) |
| B | Yeast extract with high amino acid content | 65317 | 1.01 |
| D | Yeast extract with high amino acid content | 76739 | 0.38 |
| E | Yeast extract with high nucleic acid content | 28256 | 36.26 |
| F | Yeast extract with high amino acid content and with high nucleic acid content | 44737 | 3.91 |
| H | Yeast extract with high succinic acid content | 29722 | 11.41 |
| I | Yeast extract with high glutamic acid content | 21044 | 0.55 |
| J | Amino acid, nucleic acid-containing yeast extract | 24857 | 4.76 |

Various amino acids required for the proliferation of cultured cells in DMEM were added to "A.A.-IMEM Buffer + yeast extract" according to conditions 1 to 16 in Table 3, and a cell culture test was conducted. Accordingly, the essential amino acid types that must be added as amino acids for cell proliferation when the yeast extract is used as the culture material were examined. The amount of each amino acid added was the same as that contained in the formulation of DMEM. In addition, the same amino acid composition as that of DMEM was used as P.C. (positive control), while the same amino acid composition as that of DMEM but with 1/8^{th} the amount added was used as condition 19, and that with 1/32^{nd} the amount added was used as condition 20. All reagents used in the experiment were of food additive grade.

**Table 3 Amino acid conditions for test media**

| [Table 3] | | | |
|---|---|---|---|
| | Substance name | mg/L | Reagent manufacturer |
| Condition 1 | No amino acid added | - | - |
| Condition 2 | L-Isoleucine | 107 | Nippon Rika Co., Ltd. |
| Condition 3 | L-Leucine | 107 | Nippon Rika Co., Ltd. |
| Condition 4 | L-Threonine | 97 | Nippon Rika Co., Ltd. |
| Condition 5 | L-Tryptophan | 16 | Nippon Rika Co., Ltd. |
| Condition 6 | L-Valine | 96 | Nippon Rika Co., Ltd. |
| Condition 7 | L-Histidine | 32 | Junsei Chemical Co., Ltd. |
| Condition 8 | L-Phenylalanine | 67 | Nippon Rika Co., Ltd. |
| Condition 9 | L-Methionine | 30 | Nippon Rika Co., Ltd. |
| Condition 10 | L-Lysine hydrochloride | 149 | Nippon Rika Co., Ltd. |
| Condition 11 | L-Tyrosine | 73 | Nippon Rika Co., Ltd. |
| Condition 12 | L-Arginine | 71 | KANTO CHEMICAL CO., INC. |
| Condition 13 | Glycine | 30 | Junsei Chemical Co., Ltd. |
| Condition 14 | L-Serine | 43 | Frontierfoods, INC. |
| Condition 15 | L-Cystine | 49 | Nippon Rika Co., Ltd. |
| Condition 16 | L-Glutamine | 596 | Nippon Rika Co., Ltd. |
| Condition 17 | Amino acids other than the above L-tryptophan were added | 1548 | - |
| Condition 18 | Amino acids other than the above L-glutamine were added | 968 | - |
| Condition 19 | 1/8^{th} the amount of all amino acids above was added | 196 | |
| Condition 20 | 1/32^{nd} the amount of all amino acids above was added | 49 | - |
| P.C. | All of the above amino acids were added. | 1564 | - |

10% heat-inactivated fetal bovine serum (FBS) and 1% penicillin-streptomycin-amphotericin (PSA) were further added to A.A.-IMEM Buffer + yeast extract and the amino acids of each condition listed in Table 3, so as to conduct a cell culture test.

For the cell culture test, mouse-derived myoblast cell line C2C12 was used. C2C12 cells were seeded in 12-well plates at 5 × 10⁴ cells/well and cultured in a CO₂ incubator at 37°C for 3 days in the medium under each condition. After culture under each condition, the cells were observed for adhesion and proliferation. Furthermore, images of the cells after culture were taken to confirm the state of cell growth.

### (Result)

The results of the cell images are shown in Fig. 1. From the cell images in Fig. 1, the results are shown in Table 4, such that a condition where 50% or more of the bottom surface of the culture vessel covered by cells was marked with O, a condition where 10% to 50% of the bottom surface of the culture vessel covered by cells was marked with Δ, and a condition where no cell adhesion observed was marked with ×.

**Table 4 Cell proliferation score under each condition**

| [Table 4] | | | | | |
|---|---|---|---|---|---|
| Condition 1 | Condition 2 | Condition 3 | Condition 4 | Condition 5 | Condition 6 |
| × | × | × | × | × | × |

| Condition 7 | Condition 8 | Condition 9 | Condition 10 | Condition 11 | Condition 12 |
|---|---|---|---|---|---|
| × | × | × | × | × | × |

| Condition 13 | Condition 14 | Condition 15 | Condition 16 | Condition 17 | Condition 18 |
|---|---|---|---|---|---|
| × | × | Δ | × | ○ | × |

| Condition 19 | Condition 20 | P.C. | | | |
|---|---|---|---|---|---|
| ○ | × | ○ | | | |

As shown in Fig. 1 and Table 4, when all amino acids were added (P.C.) and when 1/8^{th} the amount was added (condition 19), cell adhesion and subsequent proliferation were observed; however, when 1/32^{nd} the amount of all amino acids was added (condition 20) or when no amino acid was added (condition 1), cell adhesion was not observed. This revealed that the yeast extract (final concentration in the medium: 1 g/L) alone does not provide enough amino acids for cell adhesion and proliferation, and that further addition of amino acids is necessary.

The amino acids required in DMEM medium were added individually to examine which amino acids were required to be added in addition to the yeast extract. As a result, cell adhesion was not observed when most amino acids were added individually; however, cell adhesion was observed only under condition 15, in which L-cystine was added, making it clear that cell adhesion is improved by adding L-cystine to the yeast extract.

Furthermore, when at least 1/8^{th} the amount of all amino acids was added (condition 19, P.C.), the cells proliferated and adhered, whereas the percentage of cells that adhered was significantly lower under condition 18, in which only L-glutamine was excluded, finding that L-glutamine is necessary for cell proliferation. On the other hand, under condition 16, in which only L-glutamine was added, no cell adhesion was observed, proving that L-glutamine alone is insufficient and that L-cystine is also necessary. These results revealed that the minimum amino acids required for a basal medium are L-cystine and L-glutamine.

Moreover, cell adhesion and cell proliferation were confirmed under condition 19, whereas almost no cells adhered under condition 20, suggesting that at least 6.13 mg/L L-cystine and 74.5 mg/L L-glutamine are required.

### Example 2 Culture test for multiple cell types

In Example 1, it was demonstrated that the addition of L-cystine and L-glutamine is necessary for culturing C2C12 cells. In this example, it was verified whether these amino acids are essential not only for C2C12 cells, which are of a mouse-derived myoblast cell line, but also for other established cell lines. A cell culture test was conducted in the same manner as in Example 1 using MDBK cells (a bovine kidney-derived cell line) and HDFa cells (an adult human dermal fibroblast cell line).

### (Method)

Of the constituents of DMEM, those excluding amino acids was dissolved in ion-exchanged water to obtain A.A.-IMEM Buffer (Table 1), and each yeast extract used in Example 1 was blended in equal amounts to obtain a yeast extract Mix. This yeast extract Mix was further added so that the final concentration in the medium was 1 g/L. L-Cystine or L-cystine and L-glutamine were added thereto as shown in Table 5, and the growth of each cell was confirmed. The cell culture test was conducted by further adding 10% heat-inactivated fetal bovine serum (FBS) and 1% penicillin-streptomycin-amphotericin (PSA) to the A.A.-IMEM Buffer + yeast extract and the amino acids under each condition in Table 5.

**Table 5 Amino acid conditions for test media**

| [Table 5] | | | |
|---|---|---|---|
| | Substance name | mg/L | Reagent manufacturer |
| Condition 1 | No amino acid added | - | - |
| Condition 2 | L-Cystine | 49 | Nippon Rika Co., Ltd. |
| Condition 3 | L-Cystine | 49 | Nippon Rika Co., Ltd. |
| | L-Glutamine | 596 | Nippon Rika Co., Ltd. |
| P.C. | All amino acids listed in Table 2 | 1564 | - |

MDBK cells and HDFa cells were each seeded in 12-well plates at 5 × 10⁴ cells/well and cultured in a CO2 incubator at 37°C. The state of MDBK cells was photographed on day 5 of culture and that of HDFa cells was photographed on day 4 of culture to confirm the adhesion state of the cells.

### (Result)

The results of the cell images are shown in Fig. 2. From the cell images in Fig. 2, the results are shown in Table 6, such that a condition where 50% or more of the bottom surface of the culture vessel covered by cells was marked with O, a condition where 10% to 50% of the bottom surface of the culture vessel covered by cells was marked with △, and a condition where no cell adhesion observed was marked with ×.

**Table 6 Cell proliferation score under each condition**

| [Table 6] | | | | |
|---|---|---|---|---|
| | Condition 1 | Condition 2 | Condition 3 | P.C. |
| MDBK cells | × | ○ | ○ | ○ |
| HDFa cells | × | Δ | ○ | ○ |

As shown in Fig. 2 and Table 6, no cell adhesion was observed in any of the cells under condition 1, where no amino acids were added and only yeast extract Mix was added. In contrast, cell adhesion was observed under condition 2, where L-cystine was added. Furthermore, cell proliferation was confirmed under condition 3, where L-cystine and L-glutamine were added.

These results confirmed that L-cystine and L-glutamine contribute greatly to cell adhesion and proliferation. It became clear that the inclusion of L-cystine, L-glutamine, and the yeast extract in the medium makes it possible to culture at least the three types of established cell lines examined in Examples 1 and 2.

### Example 3 Examination of yeast extract to be added - Test to confirm suitability of various yeast extracts for cell proliferation

In this example, in order to select the optimum yeast extract to be added to the medium, a test to confirm suitability for cell proliferation was conducted using multiple yeast extracts with different characteristics.

### (Method)

The basal medium had the formulation shown in Table 7.

**Table 7 Test basal medium**

| [Table 7] | | |
|---|---|---|
| Substance name | Concentration (mg/L) | Product manufacturer |
| Potassium chloride | 400 | Tomita Pharmaceutical Co., Ltd. |
| Sodium chloride | 6400 | Tomita Pharmaceutical Co., Ltd. |
| Glucose | 4500 | Kanematsu Ensho Co., Ltd. |
| L-Glutamine | 584 | Frontierfoods, INC. |
| L-Cystine | 48.34 | Frontierfoods, INC. |
| Sodium bicarbonate | 3700 | Tokuyama Corporation |

Any one of eight types of yeast extracts A to H in Table 8 were added to media so that the final concentrations in the media were 0.25 g/L, 0.5 g/L, 1.0 g/L, or 2.0 g/L. All eight types of yeast extracts were derived from the genus *Saccharomyces* and had the characteristics shown in Table 8.

**Table 8 Types of yeast extracts used**

| [Table 8] | | | |
|---|---|---|---|
| | Characteristic of yeast extract | Total amount of amino acids (mg/100g) | Total amount of nucleic acids (%) |
| A | Yeast extract with high amino acid content | 48231 | 0.16 |
| B | Yeast extract with high amino acid content | 65317 | 1.01 |
| C | Yeast extract with high amino acid content | 67756 | 2.32 |
| D | Yeast extract with high amino acid content | 76739 | 0.38 |
| E | Yeast extract with high nucleic acid | 28256 | 36.26 |
| F | Yeast extract with high amino acid content and with high nucleic acid content | 44737 | 3.91 |
| G | Yeast extract with high nucleic acid content | 27000 | 33.77 |
| H | Yeast extract with high succinic acid content | 29722 | 11.41 |

For the test, primary cultured cells, duck liver-derived cells, were used. After extraction from the living body, the duck liver-derived cells were cultured in a medium prepared by adding 10% serum and 1% PSA to a basal medium having the same formulation as that of DMEM, up to the 3^{rd} passage. The duck liver-derived cells were seeded in 96-well plates at 1.5 × 10⁴ cells/well, 10% serum and 1% PSA were added to each medium, and a cell culture test was conducted.

Cell proliferation was recorded using an Incucyte SX5 (Sartorius). Specifically, the percentage of an area in an observation image covered by cells; that is, cell proliferation in each medium, was recorded as the cell confluence area (%) over time. The recorded data were fitted with a logistic curve, the midpoint of cell proliferation (Exponential Growth 50%: EG50) was calculated, and the cell proliferation rates were compared. A yeast extract with which cells took more than 100 hours to reach EG50 were determined that it was capable of inhibiting cell proliferation.

DMEM (Fujifilm) was used as P.C., and the basal medium described in Table 6 without yeast extract added was used as N.C.

### (Result)

The results of the time to reach EG50 of cells cultured in each medium are shown in Table 9. Comparing the EG50 in Table 9, it was found that the cell proliferation ability was reduced when yeast extract B, yeast extract E, or yeast extract F was added at a high concentration of 2.0 g/L or 1.0 g/L. However, these proliferation inhibitions were reduced by lowering the concentration. Furthermore, even when yeast extract F, yeast extract H, and yeast extract G, which is not capable of inhibiting cell proliferation when added at a high concentration, were added at 0.5 g/L or less, the time to reach EG50 was 100 hours or more, and the cell proliferation rate was reduced. These results revealed that these yeast extracts suppress cell proliferation even at low concentrations.

**Table 9 Time to reach EG50 (h) of cells cultured in each medium**

| [Table 9] | | | | | | | | | | |
|---|---|---|---|---|---|---|---|---|---|---|
| Amount of yeast extract added (g/L) | P.C. | N.C. | A | B | C | D | E | F | G | H |
| 2.0 | | | 63.15 | 200.12 | 47.57 | 23.34 | 31757.88 | 248749931 | 88.81 | 111.91 |
| 1.0 | 25.5 | 27.2 | 74.37 | 424.35 | 96.15 | 32.00 | 667.98 | 981.75 | 99.20 | 124.44 |
| 0.5 | | | 67.75 | 64.38 | 30.44 | 61.87 | 51.45 | 820.82 | 584.51 | 428.71 |
| 0.25 | | | 57.61 | 42.14 | 56.49 | 56.93 | 42.75 | 54.67 | 350.86 | 140.55 |

### Example 4 Examination of yeast extract to be added - Selection of optimal yeast extract

In this example, an experiment was carried out to select the yeast extract most suitable for cell proliferation. The yeast extracts used in the selection were yeast extracts A, B, C, and D, excluding the yeast extracts found in Example 3 to be capable of inhibiting cell proliferation at low concentrations.

### (Method)

Primary cultured cells, duck liver-derived cells were used in the experiment. After extraction from the living body, the duck liver-derived cells were cultured up to the 5^{th} to 6^{th} passages in a basal medium having the same formulation as that of DMEM, but supplemented with 10% serum and 1% PSA. The cells were repeatedly subcultured to examine whether they could proliferate.

Each yeast extract was added to the test basal medium in Table 7, so that the final concentration in the medium was 1.0 g/L, and 10% serum and 1% PSA were further added to make a complete medium.

Duck liver-derived cells were seeded in 60 mm dishes at 1.5 × 10⁵ cells/dish and cultured. At the time of passage, a portion of the cells was seeded in a 96-well plate at 5 × 10³ cells/well, and then the number of viable cells was compared by measuring absorbance at 450 nm using Cell Counting Kit-8 (Dojindo Laboratories). Culture was performed up to the 3^{rd} passage, and the number of viable cells when cultured with each yeast extract was compared based on the absorbance value.

### (Result)

In the N.C. (negative control) to which no yeast extract was added, the cells did not proliferate, and they could no longer be cultured at the 2^{nd} passage. On the other hand, it was revealed that the duck liver-derived cells proliferated even by subculture in all media containing yeast extracts added thereto. It was also confirmed that the cells proliferated more in the media containing particularly yeast extract A, yeast extract B, and yeast extract C added thereto than the media containing other yeast extracts added thereto (Fig. 3).

### Example 5 Optimization of yeast extract-added medium

In this example, yeast extracts A and B, which were found to be particularly effective in cell proliferation in Example 5, were used to examine the optimum medium composition.

### (Method)

Primary cultured cells, duck liver-derived cells were used in the experiment. After extraction from the living body, the duck liver-derived cells were cultured up to the 5^{th} to 6^{th} passages in a basal medium having the same formulation as that of DMEM but supplemented with 10% serum and 1% PSA. The cells were repeatedly subcultured to examine whether they could proliferate.

To the test basal medium in Table 10, 2.0 g/L yeast extract each was added, and 10% serum and 1% PSA were added to make a complete medium. In this test, calcium chloride was further added to the formulation of the basal medium in Table 7 of Example 4 in an amount equal to the amount of calcium chloride contained in DMEM, and the test was conducted (Table 10).

**Table 10 Test basal medium**

| [Table 10] | | |
|---|---|---|
| Substance name | Concentration (mg/L) | Product manufacturer |
| Potassium chloride | 400 | Tomita Pharmaceutical Co., Ltd. |
| Sodium chloride | 6400 | Tomita Pharmaceutical Co., Ltd. |
| Calcium chloride | 378.44 | Tomita Pharmaceutical Co., Ltd. |
| Glucose | 4500 | Kanematsu Ensho Co., Ltd. |
| L-Glutamine | 584 | Frontierfoods, INC. |
| L-Cystine | 48.34 | Frontierfoods, INC. |
| Sodium bicarbonate | 3700 | Tokuyama Corporation |

Duck liver-derived cells were seeded in 60 mm dishes at 1.5 × 10⁵ cells/dish and cultured. At the time of passage, a portion of the cells was seeded in a 96-well plate at 5 × 10³ cells/well, and the number of viable cells was compared by measuring absorbance at 450 nm using Cell Counting Kit-8 (Dojindo Laboratories). Culture was performed up to the 5^{th} passage, and the number of viable cells when cultured with each yeast extract was compared as the absorbance value.

For P.C. (positive control), a medium having the same formulation as that of the DMEM was used, and for N.C. (negative control), only the test basal medium having the formulation shown in Table 10, containing no yeast extract added was used.

### (Result)

The results are shown in Fig. 4. It was revealed that culturability is improved by increasing the amount of the yeast extract added to 2.0 g/L and adding calcium chloride to the basal medium. In N.C., cells stopped proliferation at the 3^{rd} passage and became unable to be cultured thereafter, but it was confirmed that about 70% or more of the culturability of P.C. can be maintained at the 5^{th} passage in the medium containing the yeast extracts added thereto.

### Example 6 General-purpose confirmation test 1 of yeast extract-added medium

In this example, yeast extract A, which was found to exhibit particularly good cell proliferation in Example 5, and yeast extract A-B, which was a blend of equal amounts of A and B, were used to conduct a culture test on cells derived from various chicken organs, and the general-purpose confirmation test was conducted for these yeast extracts.

### (Method)

In the experiment, primary cultured cells, cells derived from chicken liver, muscle, lung, kidney and brain were used. After extraction from the living body, cells derived from various chicken organs were then cultured in each medium. The formulation of the yeast extract-added medium used was prepared by adding vitamins contained in DMEM (calcium pantothenate, folic acid, pyridoxyl hydrochloride, riboflavin, thiamine hydrochloride, glycerophosphatidylcholine, myo-inositol and nicotinamide in Table 1) to the test basal medium having the formulation in Table 10 of Example 5 in the same amounts as those of the formulation of DMEM, and adding 2.0 g/L yeast extract A. In addition, a basal medium having the same formulation as that of DMEM was used as a positive control. These media supplemented with 10% serum and 1% PSA were used in the test.

Cells derived from various organs extracted from chickens were seeded in each of yeast extract-added media and the positive control medium, and passaged every 3 to 4 days, and the number of cells was counted at the 3^{rd} passage. The cells were seeded in 6-well plates to give the number of cells seeded at the time of passage at 3 × 10⁵ cells/well.

### (Result)

The results are shown in Fig. 5. For all cells derived from various chicken organs, it was confirmed that the cells cultured in the yeast extract A-added medium grew at the same level as the positive control. This revealed that the yeast extract-added medium can maintain good culturability for cells derived from various organs. Furthermore, the yeast extract A-B-added medium resulted in slightly lower culturability in the cases of lung, kidney, and brain, but it was observed that it had culturability in the cases of liver and muscle at the same level as those of the P.C. and yeast extract A-added medium.

### Example 7 General-purpose confirmation test 2 of yeast extract-added medium

In this example, using yeast extract A found to have excellent proliferation ability in Example 6, a culture test was conducted on established cell lines and primary cultured cells of different origins to confirm culturability.

In the experiment, established cell line RL34 (cells derived from normal rat liver), MDBK (cells derived from normal bovine kidney), and primary cultured cells, bovine muscle-derived cells were used. The formulation of a yeast extract-added medium used was prepared by adding vitamins (calcium pantothenate, folic acid, pyridoxyl hydrochloride, riboflavin, thiamine hydrochloride, glycerophosphatidylcholine, myo-inositol, and nicotinamide in Table 1) contained in DMEM to a basal medium having the formulation shown in Table 10 of Example 5, in the same amounts as in the formulation of DMEM, and adding 2.0 g/L yeast extract A. DMEM was used as a positive control. These media supplemented with 10% heat-inactivated FBS and 1% PSA were used in the test.

### (Method)

Each type of cells cultured in DMEM was seeded in 6-well plates; specifically, RL34 cells were seeded at 3 × 10⁵ cells, MDBK cells were seeded at 2 × 10⁵ cells, and bovine muscle-derived cells were seeded at 3 × 10⁵ cells. The culture test was conducted in each medium. Subculture was performed every 3 to 4 days, and the number of cells was counted.

### (Result)

The results are shown in Figs. 6 to 8. It was confirmed that all the cells cultured in the yeast extract A-added media maintained 70% or more of the culturability compared to that of the cells cultured in the positive control DMEM.

### Example 8 Examination of the lowest limit range of the concentration of amino acids added to basal medium

In this example, the concentration range of amino acids (L-glutamine, L-cystine) added to the basal medium, particularly the minimum necessary concentration, was examined.

### (Method)

MDBK cells cultured in DMEM were seeded in 12-well plates at 1 × 10⁵ cells/well, and medium was added at 2 ml/well to culture the cells. Subculture was performed every 3 to 4 days, and the number of viable cells was measured at the end of the third subculture.

In addition, each medium supplemented with heat-inactivated FBS 10% and PSA 1% was used in the test.
Cells used: MDBK cells (established cell line)
Number of cells before culture: 1 × 10⁵ cells
   Concentration range of amino acids added: 0.002 to 0.063% by mass 0.063% by mass was defined as 1, and the amount of amino acids added was reduced from 0.063% by mass in two-fold serial steps, until the lowest amount of amino acids added was 0.002% by mass. The formulations of the basal media are shown in the table below.

**[Table 11]**

| Substance name | 9 | | | | | |
|---|---|---|---|---|---|---|
| | ×1 | ×1/2 | ×1/4 | ×1/8 | ×1/16 | ×1/32 |
| Potassium chloride | 0.4 | 0.4 | 0.4 | 0.4 | 0.4 | 0.4 |
| Sodium chloride | 6.4 | 6.4 | 6.4 | 6.4 | 6.4 | 6.4 |
| Glucose | 4.9 | 4.9 | 4.9 | 4.9 | 4.9 | 4.9 |
| **L-Glutamine** | **0.590** | **0.295** | **0.148** | **0.074** | **0.037** | **0.018** |
| **L-Cystine** | **0.049** | **0.025** | **0.012** | **0.006** | **0.003** | **0.002** |
| Sodium bicarbonate | 3.7 | 3.7 | 3.7 | 3.7 | 3.7 | 3.7 |
| Yeast extract A | 2 | 2 | 2 | 2 | 2 | 2 |
| Calcium chloride | 0.38 | 0.38 | 0.38 | 0.38 | 0.38 | 0.38 |
| Calcium pantothenate | 0.004 | 0.004 | 0.004 | 0.004 | 0.004 | 0.004 |
| α-GPC | 0.0074 | 0.0074 | 0.0074 | 0.0074 | 0.0074 | 0.0074 |
| Folic acid | 0.004 | 0.004 | 0.004 | 0.004 | 0.004 | 0.004 |
| myo-Inositol | 0.0072 | 0.0072 | 0.0072 | 0.0072 | 0.0072 | 0.0072 |
| Nicotinamide | 0.004 | 0.004 | 0.004 | 0.004 | 0.004 | 0.004 |
| Pyridoxine hydrochloride | 0.004 | 0.004 | 0.004 | 0.004 | 0.004 | 0.004 |
| Riboflavin | 0.0004 | 0.0004 | 0.0004 | 0.0004 | 0.0004 | 0.0004 |
| Thiamine hydrochloride | 0.004 | 0.004 | 0.004 | 0.004 | 0.004 | 0.004 |
| Water | 1000 | 1000 | 1000 | 1000 | 1000 | 1000 |
| Whole culture medium, amino acid content percentage (w/v, %) | 0.063% | 0.031% | 0.016% | 0.008% | 0.004% | 0.002% |
| Whole culture medium, L-glutamine (w/v, %) | 0.058% | 0.029% | 0.014% | 0.007% | 0.004% | 0.002% |
| Whole culture medium, L-cystine (w/v, %) | 0.0048% | 0.0024% | 0.0012% | 0.0006% | 0.0003% | 0.0002% |
| Amino acid content percentage, in terms of solid content (w/w, %) | 3.46% | 1.73% | 0.87% | 0.43% | 0.22% | 0.11% |
| L-Glutamine, in terms of solid content (w/w, %) | 3.20% | 1.60% | 0.80% | 0.40% | 0.20% | 0.10% |
| L-Cystine, in terms of solid content (w/w, %) | 0.27% | 0.13% | 0.07% | 0.03% | 0.02% | 0.01% |

The results of cell proliferation when cultured in the above basal media are shown in Fig. 9. The horizontal axis in Fig. 9 represents the parts by mass (w/v, %) of amino acids (L-glutamine, L-cystine) contained in the basal medium, per 100 parts by mass of the basal medium. The vertical axis in Fig. 9 represents the number of viable cells at the end of the third subculture.

### (Result)

At the 2^{nd} passage, it was confirmed that MDBK cells proliferated even when the amount of amino acids added was reduced to 0.002% by mass. Furthermore, at the 3^{rd} passage, no cell proliferation was observed at 0.002% by mass, but maintaining the cells was still possible. Furthermore, even at the 3^{rd} passage, cell proliferation was confirmed at 0.004% by mass or more.

These results indicate that the lower limit range of the concentration of the content of L-cystine and L-glutamine is 0.002% by mass based on the basal medium, the amount of which is defined as 100% by mass.

### Example 9 Confirmation of culturability when L-cystine was replaced with L-cysteine

In this example, the culturability when L-cystine was replaced with L-cysteine was confirmed.

In this example, L-cysteine hydrochloride monohydrate, which is more soluble than L-cystine, was used as an amino acid added to the basal medium, and the culturability therewith was examined as compared to that with L-cystine, when.

### (Method)

Cells used: MDBK cells (established cell line), RL34 cells (established cell line), duck liver-derived cells (primary cultured cells)
Number of cells before culture: MDBK and RL34 cells = 1 × 10⁵ cells/well, duck liver-derived cells = 1 × 10⁶ cells/10 cm dish
Amount of L-cysteine hydrochloride monohydrate added: Since L-cystine is a dimer of L-cysteine, L-cysteine hydrochloride monohydrate was added in an amount twice the number of moles of L-cystine.

The formulation of the basal medium is shown in the table below.

**[Table 12]**

| Substance name | g/L | |
|---|---|---|
| | L-Cystine | L-Cysteine hydrochloride monohydrate |
| Potassium chloride | 0.4 | 0.4 |
| Sodium chloride | 6.4 | 6.4 |
| Glucose | 4.9 | 4.9 |

| **Amino acid** | | |
|---|---|---|
| **L-Glutamine** | **0.59** | **0.59** |
| **L-Cystine** | **0.049** | **0** |
| **L-Cysteine hydrochloride monohydrate** | **0** | **0.072** |
| Sodium bicarbonate | 3.7 | 3.7 |
| Yeast extract A | 2 | 2 |
| Calcium chloride | 0.38 | 0.38 |
| Calcium pantothenate | 0.004 | 0.004 |
| α-GPC | 0.0074 | 0.0074 |
| Folic acid | 0.004 | 0.004 |
| myo-Inositol | 0.0072 | 0.0072 |
| Nicotinamide | 0.004 | 0.004 |
| Pyridoxine hydrochloride | 0.004 | 0.004 |
| Riboflavin | 0.0004 | 0.0004 |
| Thiamine hydrochloride | 0.004 | 0.004 |
| Water | 1000 | 1000 |
| Whole culture medium, amino acid content percentage (w/v, %) | 0.064% | 0.066% |
| Whole culture medium, L-glutamine content percentage (w/v, %) | 0.059% | 0.059% |
| Whole culture medium, L-cystine content percentage (w/v, %) | 0.005% | 0.000% |
| Whole culture medium, L-cysteine hydrochloride monohydrate content percentage (w/v, %) | 0.000% | 0.007% |
| Amino acid content percentage, in terms of solid content (w/w, %) | 3.46% | 3.58% |
| L-Glutamine, in terms of solid content (w/w, %) | 3.20% | 3.19% |
| L-Cystine, in terms of solid content (w/w, %) | 0.27% | 0.00% |
| L-Cysteine hydrochloride monohydrate, in terms of solid content (w/w, %) | 0.00% | 0.39% |

MDBK cells and RL34 cells were each subcultured in a complete medium prepared by adding 1% PSA and 10% FBS to the above basal medium. Duck liver-derived cells were also subcultured in a complete medium prepared by adding 1% PSA and 10% serum to the above basal medium. In both cases, subculture was performed every 3 to 4 days, and subculture was performed up to the third time.

### (Result)

The results at the end of the third subculture are shown in Fig. 10. The vertical axis in Fig. 10 represents the number of viable cells. When a culture test was conducted using established cell lines MDBK cells (established cell line) and RL34 cells, and primary cultured cells; that is, duck liver-derived cells, almost no difference in culturability due to the replacement of L-cystine with L-cysteine hydrochloride monohydrate was observed in all cases.

These results revealed that the addition of either L-cystine or L-cysteine as an amino acid has no effect on cell culture.

Furthermore, the same results were obtained when twice the amount of L-cysteine was further added (0.14% added).

### INDUSTRIAL APPLICABILITY

The present invention provides a basal medium for animal cells with reduced cost and environmental impact. By utilizing the medium and the method for culturing animal cells of the present invention, it is now possible to provide processed foods containing animal cells in a less expensive and less environmentally hazardous manner.

## Claims

1. A basal medium composition for animal cells, comprising a yeast extract, amino acids, an inorganic salt and a sugar, wherein the amino acids in the composition comprise cystine and/or cysteine, and glutamine.

2. The basal medium composition for animal cells according to claim 1, wherein the content of all amino acids in the composition is 0.002 to 1.0 parts by mass per 100 parts by mass of the basal medium composition.

3. The basal medium composition for animal cells according to claim 1 or 2, wherein the percentage of cystine and/or cysteine, and glutamine is 50% or more based on all amino acids in the composition.

4. The basal medium composition for animal cells according to any one of claims 1 to 3, wherein the content of cystine and/or cysteine, and glutamine in the composition is 0.001 to 1.0 parts by mass per 100 parts by mass of the basal medium composition.

5. The basal medium composition for animal cells according to any one of claims 1 to 4, wherein the content of cystine and/or cysteine, and glutamine in the composition is 0.1 to 5.0 parts by mass per solid content of the basal medium composition.

6. The basal medium composition for animal cells according to any one of claims 1 to 5, wherein the content of cystine and/or cysteine in the composition is 0.0001 to 0.02 parts by mass per 100 parts by mass of the basal medium composition.

7. The basal medium composition for animal cells according to any one of claims 1 to 6, wherein the content of cystine and/or cysteine in the composition is 0.005 to 1.0 parts by mass per solid content of the basal medium composition.

8. The basal medium composition for animal cells according to any one of claims 1 to 7, wherein the content of glutamine in the composition is 0.001 to 1.0 parts by mass per 100 parts by mass of the basal medium composition.

9. The basal medium composition for animal cells according to any one of claims 1 to 8, wherein the content of glutamine in the composition is 0.1 to 5.0 parts by mass per solid content of the basal medium composition.

10. The basal medium composition for animal cells according to any one of claims 1 to 9, wherein the yeast extract is an yeast extract with high amino acid content.

11. The basal medium composition for animal cells according to any one of claims 1 to 10, wherein the total amino acid content of the yeast extract is 0.75 parts by mass or more per 100 parts by mass of the basal medium composition.

12. The basal medium composition for animal cells according to any one of claims 1 to 11, wherein the yeast extract is an yeast extract with low nucleic acid content.

13. The basal medium composition for animal cells according to any one of claims 1 to 12, wherein the total nucleic acid content of the yeast extract is 2.0 parts by mass or less per 100 parts by mass of the basal medium composition.

14. The basal medium composition for animal cells according to any one of claims 1 to 13, wherein the yeast extract is an yeast extract with high amino acid content and with low nucleic acid content.

15. The basal medium composition for animal cells according to any one of claims 1 to 14, wherein the content of the yeast extract is 0.05 to 20.0 parts by mass per 100 parts by mass of the basal medium composition.

16. The basal medium composition for animal cells according to claim 1 or 2, further comprising a vitamin.

17. A method for culturing animal cells, comprising culturing animal cells in a basal medium for animal cells, wherein the basal medium comprises a yeast extract, amino acids, an inorganic salt and a sugar, and the amino acids are cystine and/or cysteine, and glutamine.

18. The method according to claim 17, wherein the yeast extract is an extract from an yeast with high amino acid content, an extract from a yeast with low nucleic acid content, or an extract from an yeast with high amino acid content and with low nucleic acid content.

19. An animal cell, which is cultured by the method according to claim 17 or 18.

20. A processed food, comprising the animal cell according to claim 19.
